(19) Europäisches Patentamt
European Patent Office
Office européen des brevets

(11) **EP 0 634 654 B1**

(12) **EUROPEAN PATENT SPECIFICATION**

(45) Date of publication and mention
of the grant of the patent:
**18.08.1999 Bulletin 1999/33**

(51) Int Cl.[6]: **G01N 33/18**, G01N 21/53,
G01N 21/85

(21) Application number: **94110344.2**

(22) Date of filing: **04.07.1994**

(54) **Device for monitoring the quality of purified water, particularly for biological purification plants**

Vorrichtung zur Kontrolle der Wasserqualität, insbesondere für biologische Kläranlagen

Dispositif de contrôle de la qualité de l'eau, en particulier pour des installation de clarification biologique

(84) Designated Contracting States:
**AT CH DE ES FR GB GR IT LI**

(30) Priority: **14.07.1993 IT MI931547**

(43) Date of publication of application:
**18.01.1995 Bulletin 1995/03**

(73) Proprietor: **Ventura, Arcangelo**
**I-25126 Brescia (IT)**

(72) Inventor: **Ventura, Arcangelo**
**I-25126 Brescia (IT)**

(74) Representative: **Modiano, Guido, Dr.-Ing.**
**Modiano & Associati SpA**
**Via Meravigli, 16**
**20123 Milano (IT)**

(56) References cited:
**EP-A- 0 361 904**      **EP-A- 0 559 305**
**WO-A-86/07454**      **DE-U- 9 216 020**
**DE-U- 9 319 750**      **FR-A- 2 635 587**

- **CHEMOMETRICS AND INTELLIGENT LABORATORY SYSTEMS, JUNE 1993, NETHERLANDS, vol. 19, no. 2, ISSN 0169-7439, pages 181-185, DANIGEL H ET AL 'Computer-controlled waste water monitoring for industrial purposes'**
- **INTERNATIONAL LABORATORY, vol. 16, no. 7, September 1986 FAIRFIELD CT US, pages 100-110, FITCH P ET AL 'Remote UV-VIS-NIR spectroscopy using fiber optic chemical sensing'**

Remarks:
The file contains technical information submitted after the application was filed and not included in this specification

## Description

The present invention relates to a device for monitoring the quality of purified water, particularly for biological purification plants.

As is known, monitoring of activated-sludge municipal purification plants is performed by measuring the functional parameters of the individual compartments of the plant and of the machines.

This measurement is performed by:

-- measuring functional data: PH, temperature, conductivity, dissolved oxygen, pump operation hours (therefore approximately 95% of the daily flow rates), aeration hours, volume of sludge in 11/30' cone (VF);

-- retrieving sludge samples: under aeration for microscopic investigations and dry content measurement (ssa); under recirculation for dry content measurement (ssr).

Measured and calculated parameters provide additional data, such as the Mohlam index (Im), recirculation ratio, sludge load (with input analyses), and settling solid load.

Occasional retrieval of samples at the input and at the output furthermore allows to determine the input load, its biodegradability, purification efficiencies, and most of all whether the output parameters comply with the applicable statutory provisions.

In normal municipal purification plants (and generally in biological activated-sludge plants), chemical analyses are performed only occasionally: in the best of cases, every week, usually every month or two if not more.

In order to obtain a more frequent indication between successive analyses of the quality of the purified water and to make the related parameters fall within the limits prescribed by the applicable statutory provisions, water monitoring is performed by considering that if the plant is running correctly, the output water is clear and quite probably the parameters are within the limits set by the law.

In terms of plant organization, the indication is obtained by measuring the "clarity" of the water and by creating a scale graduated from 0 to 10, where 0 indicates highly polluted water and 10 indicates very clear water with parameters within the limits of the law.

Known devices used to monitor sludge-based purification plants currently measure only the value of the clarity of the output water and are unable to give indications as to the presence of individual substances in the water. Furthermore, known devices require the retrieval of a sample of the output water, which is introduced in the clarity-measuring device.

In this manner it is not possible to monitor the output water continuously and without the aid of an operator.

Known devices furthermore do not indicate the probable content of the individual substances present in the water to be monitored.

A principal aim of the present invention is to eliminate the drawbacks described above by providing a device for the continuous monitoring of the quality of purified water, particularly for biological purification plants, which is capable of providing the values of the quality of the purified water and the related percentages of probability, so that the parameters of the purified water are within the limits of the law.

Within the scope of the above aim, an object of the present invention is to provide a device that provides the user with the above mentioned values and with the associated percentages in a continuous manner.

Another object of the present invention is to provide a device that can be easily installed in purification plants. Another object of the present invention is to provide a device that does not require recharging electrolytes or solutions.

Another object of the present invention is to provide a device which is highly reliable, relatively easy to manufacture and at competitive costs.

With this aim, these objects and others in view, which will become apparent hereinafter, the invention provides a device for monitoring the quality of purified water, particularly for biological purification plants, as defined in claim 1.

Further characteristics and advantages of the invention will become apparent from the following detailed description of a preferred but not exclusive embodiment thereof, illustrated only by way of non-limitative example in the accompanying drawings, wherein:

figure 1 is an electrical block diagram of the device according to the present invention;
figure 2 is an axial sectional view of the measurement probe according to the present invention;
figure 3 is a front view of the visualization means of the device according to the present invention.

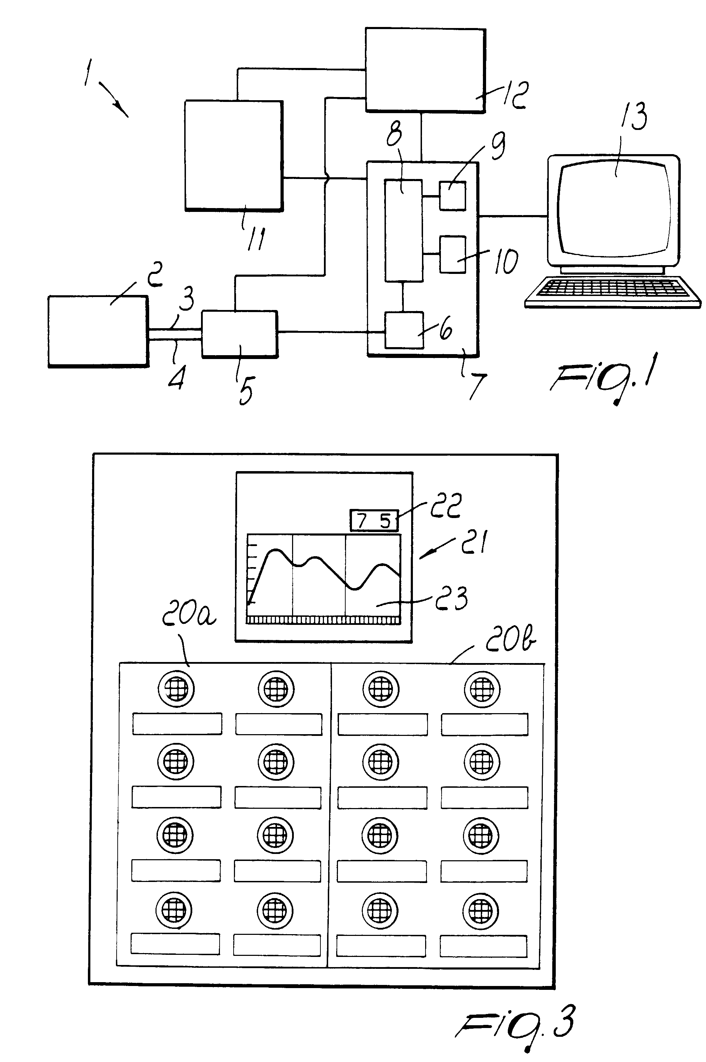

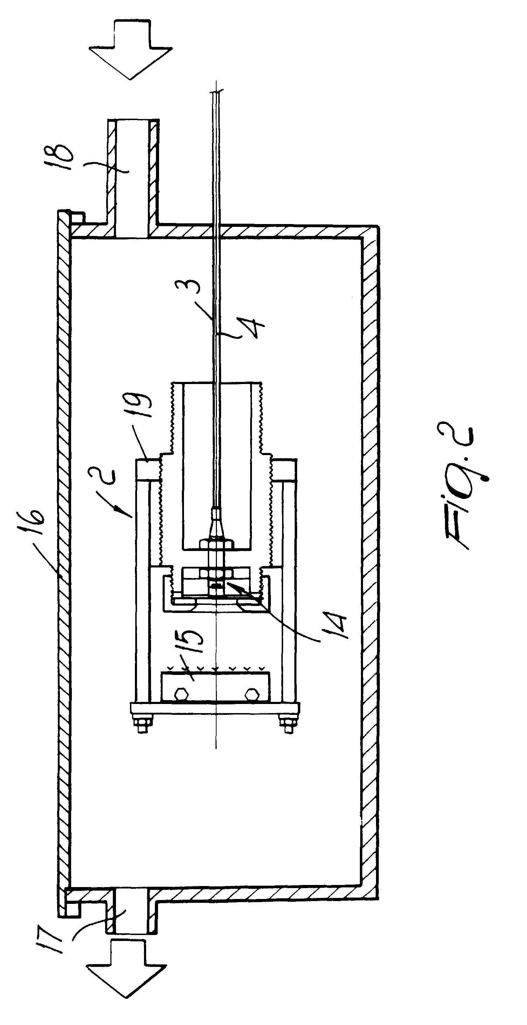

With reference to figure 1, the reference numeral 1 designates the device according to the present invention. The reference numeral 2 designates the probe for measuring the clarity of the purified water; said probe is normally immersed in the output water of the purifying plant. The output of the measurement probe is constituted by a first illuminating optical fiber 3 and by a second return optical fiber 4.

As shown in figure 2, the probe 2 comprises the two optical fibers 3 and 4, which are joined in a single input/output terminal 14. A reflector 15 is arranged in front of the terminal 14 and reflects the light signal from the first optical fiber 3 towards the inlet of the second optical fiber 4. The inclination of the reflector 15 is calibrated so as to obtain an optimum response light signal. The optical fibers 3 and 4, the terminal 14 and the reflector 15 are mounted on a rigid support 19 made of stainless steel. The probe 2 is furthermore placed in a chamber 16 that has a water inlet 17 and a water outlet 18. The chamber 16, containing the probe 2, is immersed in the water leaving the purification plant. The openings 17 and 18 allow circulation of the water inside the chamber 16 and between the terminal 14 and the reflector 15.

The two optical fibers 3 and 4 are connected to a photocell unit 5, which emits the light signal, by means of a built-in fluorescent lamp, for the first optical fiber 3 and measures the signal arriving from the second optical fiber 4. The photocell unit 5 furthermore converts the difference between the emitted light signal and the received light signal into an analog DC signal on the order of 4-20 mA, according to the difference measured. This difference between the light signals is proportional to the clarity of the water in which the measurement probe is immersed.

The output of the photocell unit 5 is sent to an analog-digital converter 6 which is included in monitoring means 7. The converter 6 converts the analog signal arriving from the photocell unit 5 into a digital signal that can be read by a microprocessor 8 of the monitoring means 7. The microprocessor is furthermore provided with a random-access memory 9 and with a wave-shaping circuit 10.

Visualization means, constituted by a panel 11 provided with LEDs and with an X-Y recorder, are driven by the monitoring means 7.

A power supply unit 12, which is supplied at mains voltage, is connected to the photocell unit 5, to the monitoring means 7 and to the visualization panel 11 so that they are supplied with low-voltage power.

Finally, an external computer 13 is connected to the monitoring means 7. Such computer may be an IBM PC compatible computer which has an Intel 80386 processor and 2Mb of RAM, an interace card. The computer 13 may be supplied with a printer which prints the required data (plant, year, month, day, hour, minutes, % of probability, graphs). Such data may also be stored on the hard disk or diskettes of the PC 13.

The microprocessor 8, or the computer 13, provide the data related to probability that the purified water fall within the limits set by the applicable statutory provisions, based on clarity reading.

In order to set up the microprocessor 8 and/or the computer 13, clarity readings have been correlated with various sample dilutions for a given number of purified water samples, obtaining, on a logarithmic scale, an interpolated straight line of the form:

$$Y = 2a - a \log. X \qquad (1)$$

where Y is the percentage of dilution, X is the clarity reading of the sample, and $\underline{a}$ is the gradient of the straight line.

From the above equation it has been possible to obtain the function that provides the reading value X1 of a sample that is diluted at the percent concentration Y1, assuming that it initially had a clarity value Xo. The equation is in fact:

$$X1 = Xo + K \left(10^{-Y1/a} - 10^{-Yo/a}\right) \qquad (2)$$

where X1 is the clarity value calculated for a sample diluted/concentrated at the percent concentration of Y1; Xo is the initial clarity reading; K is the constant, which is equal to the reference clarity value; Y1 is the ideal sample concentration for clarity X1; and Yo is the ideal sample concentration for clarity Xo.

This last equation has the purpose of correlating the various clarity readings Xo measured on the samples with the reading X1 related to the sample diluted or concentrated by the factor Y1.

For example, the clarity of a sample of water is measured as Xo = 60. Laboratory analyses show that BOD5 substance is equal to 50 ppm. In order to comply with Table A of Italian Law no. 319/76, BOD5 must be equal to 40 ppm. If the sample is diluted at the ratio 40:50 = 80% (80 cc of sample in 100 cc), one obtains the reading X1 = 66.13 (calculated with equation (2)).

It is necessary to perform these checks on a large number of water samples (more than 200) in order to obtain water clarity values X1 regarding the most significant parameters, such as: sedimentation solids, suspended solids, BOD5, COD, total reduced nitrogen Kj, ammonia nitrogen, total phosphorus, MBAS surfactants, nitrates, etc.

For input samples, since clarity readings are close to zero, it is necessary to perform dilutions and measure clarity, checking that the value Xo is close to zero by means of equation (2).

The values obtained are processed in order to obtain frequency groups and average values. The curves of equations (1) and (2) allow to determine the percentages of probability that the water, for a clarity reading X, is within the limits of the law (Table A, Law no. 319/76).

The following table shows an example of the percentages of probability for a group of plants.

| MEASURED CLARITY VALUES | | | | | | | | | |
|---|---|---|---|---|---|---|---|---|---|
| PARAMETERS | | | | | | | | | |
| | 20 | 30 | 40 | 50 | 60 | 70 | 80 | 90 | 95 |
| SEDIM. SOLIDS | 0 | 57 | 71 | 71 | 86 | 98 | | | |
| SUSP. SOLIDS | 54 | 54 | 62 | 89 | 91 | 92 | 94 | 98 | |
| BOD5 | 15 | 15 | 23 | 38 | 54 | 77 | 98 | | |
| COD | 15 | 38 | 38 | 77 | 92 | 98 | | | |
| TOTAL NITR. Kj | 86 | 98 | | | | | | | |
| AMMONIA NITROGEN | 31 | 31 | 38 | 38 | 38 | 62 | 92 | 98 | |
| TOTAL PHOSPHORUS | 58 | 67 | 75 | 83 | 83 | 83 | 83 | 98 | |
| MBAS | 77 | 85 | 98 | | | | | | |

The clarity value of 95 is the value for distilled water.

The table calculated and stored by the microprocessor 8 and/or computer 13 allows to obtain the percentages of probability for the individual substances: if the clarity reading is 50, there is a 38% possibility that BOD5 is within Table A of Law no. 316/76, a 77% probability for COD, and 98% for total reduced nitrogen Kj (TOTAL NITR. Kj).

In this manner, the microprocessor 8 can determine the percentages of probability that the concentration of various parameters in water in which the measurement probe 2 is immersed be within a preset threshold.

The visualization means 11, shown in figure 3, include a plurality of light-emitting diodes (LED), divided into two sections 20a and 20b. Each diode of each section corresponds to a particular substance for which the probability percentage is obtained, so that each substance has two diodes divided between the two sections 20a and 20b. The diodes of the section 20a visualize that, for a particular substance, the percent of probability to have a concentration within a preset threshold is lower than a preset probability limit, whereas the diodes of the section 20b visualize that, for a particular substance, such percent of probability exceeds the preset probability limit. The diodes of the two sections preferably have different colors.

A recorder 21 is included in the visualization means 11. The recorder 21 comprises a digital display 22 which displays an overall percentage (the "grade") for the purified water. The recorder furthermore has an X-Y display 23 that can display the trend of this overal percentage over time.

Alternatively, such results may be displayed on the screen of the computer 13.

Operation of the device according to the present invention is as follows. The measurement probe 2, together with its chamber 16, is immersed in the water to be monitored of the purification plant (typically at a depth between 15 and 100cm). Due to the current, the water of the purification plant is forced into the chamber 16 of the measurement probe by means of the inlet 17. In the same manner, the water can freely flow out of the chamber 16 through the outlet 18, thus allowing the water to be monitored to circulate inside the chamber 16 and between the reflector 15 and the terminal 14 of the two optical fibers.

The photocell unit sends, by means of the built-in fluorescent lamp, a light beam over the first optical fiber 3. The light beam is reflected by the reflector 15 and returns to the photocell unit 5 over the second optical fiber 4.

The reflected light beam is weaker than the transmitted beam, since the particles suspended and dissolved in the water interfere and scatter part of the light beam. The reflected beam that returns to the photocell unit 5 is thus weaker than the emitted one. The amount of scattered light depends on the size, concentration and composition of the dissolved or suspended particles that are present.

The difference between the emitted light beam and the reflected one is measured by the unit 5 and converted into a DC signal. This signal is sent to the A/D converter 6, which converts it into a numeral corresponding to the clarity of the measured liquid, which is comprised between a minimum value and a maximum value that are set on a scale chosen at will (for example from 0 to 100).

The numeral is then compared with the values of the above listed table, calculated by the microprocessor 8 and stored in the memory 9.

It should be stressed that the above listed table is only an example of a possible table based on a certain number of samples and plants. An important aspect of the invention is that it is possible to update the table continuously, either by means of the computer 13 or directly by means of the microprocessor 8, with new data from new analyses, making the reliability of the calculated estimate concentration approach 100% ever more closely.

The monitoring means 7 then switch on the diodes of the visualization means 11 as a function of the reliability percentage measured from water clarity.

The monitoring means 7 are set so that the diodes of section 20b switch on only when the probability percentages exceed a certain threshold or preset probability limit (for example 90%). The thresholds are set according to the accuracy (reliability) of the table stored in the memory 9. The accuracy of the table, i.e. the closeness of the reliability percentages to 100%, is improved by updating with data from new analyses, as described above. Furthermore, by checking clarity readings against analyses it is possible to build probability percentage curves for the individual substances for each plant, increasing reliability of said readings.

If the probability percent that the concentration of a particular substance be within a preset threshold is lower than a preset probability limit, the appropriately provided diode of the section 20a switches on; if such probability percent for a particular substance instead exceeds the preset probability limit, the related diode of the section 20b switches on.

Furthermore, the microprocessor 8 or the computer 13 calculates a single water reliability percentage which is displayed on the digital display 22 of the recorder. This numeral is considered as the quality "grade" assigned to the water to be monitored.

Finally, the X-Y display 23 visualizes the trend of this overall percentage (the "grade") over time.

The quality of the water can be measured continuously, obtaining round-the-clock monitoring.

The invention thus conceived is susceptible to numerous modifications and variations, all of which are within the scope of the inventive concept.

For example, the probe may be supplied with pneumatic cleaning means which may clean the interior of the chamber 16 and in particular the space between the reflector 15 and the two optical fibers 3 and 4.

The device according to the present invention can be used in other applications, such as the continuous monitoring of lakes and rivers.

Finally, all the details may be replaced with other technically equivalent ones.

In practice, the materials employed, as well as the shapes and dimensions, may be any according to the requirements without thereby abandoning the scope of the protection of the following claims.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Device for monitoring the quality of purified water, particularly for biological purification plants and the like, which comprises

   - measuring means (2) constituted by a measurement probe comprising means (3) for illuminating said water to be monitored, means (15) for reflecting the light emitted through said illumination means (3) and means (4) for receiving the light reflected by said reflection means (15),
   - reading means (5) suitable to generate a signal that is proportional to the difference between a light signal emitted through said illumination means and the light signal received through said receiving means after reflection by said reflection means, said reading means (5) being connected to said illumination means (3) and said receiving means (4), and
   - monitoring means (7) connected to said reading means and suitable to process the signal generated by said reading means to calculate an estimate of the concentration of noxious substances in the water to be monitored, to indicate the probabilities that the corresponding actual concentration values for said noxious substances be lower than a preset threshold and to signal if said probabilities are lower or higher than preset probability limits.

2. Device according to claim 1, characterized in that said monitoring means (7) comprise an analog-digital converter (6) and processing means (8), said convertor (6) converting the signal originating from said reading means (5) into a signal that can be read by said processing means (8).

3. Device according to claim 2, characterized in that said processing means (8,13) are suitable to process said signal generated by the convertor (6) by using equations obtained by correlation of values of said signal generated by said reading means (5) with concentration data obtained by laboratory analyses.

4. Device according to claim 3, characterized in that said equations used by said processing means (8,13) are continuously updated with new data obtained by laboratory analyses.

**5.** Device according to one or more of the preceding claims, characterized in that it comprises visualization means (11) which are associated with said monitoring means (7).

**6.** Device according to claim 5, characterized in that said visualization means (11) comprise a recorder (21) with a disply (22).

**7.** Device according to claims 5 or 6, characterized in that said visualization means (11) comprise a first section of diodes (20b) and a second section (20a) of diodes, each diode of said sections corresponding to a substance which is present in the water to be monitored, each one of said diodes of said first section (20b) being suitable to visualize that for said substance said probability that the actual concentration be within a given threshold exeeds a preset probability limit, and each one of said diodes of said second section (20a) being suitable to visualize that for said substance said probability that the actual concentration be within a given threshold is lower than a preset probability limit.

**8.** Device according to one or more of the preceding claims, characterized in that said measuring means (2) are inserted in a chamber (16) suitable to be immersed in said water to be monitored, said chamber comprising at least one inlet opening (18) and one outlet opening (17), said openings allowing the circulation of said water to be monitored between said illumination (3) and receiving means (4) and said reflection means (15).

**9.** Device according to one or more of the preceding claims, characterized in that said illumination means (3) and said means (4) for receiving the reflected light are constituted by optical fibers.

**10.** Device according to one or more of the preceding claims, characterized in that said reading means (5) comprise a photocell.

**11.** Device according to one or more of the preceding means, characterized in that said reflection means reflect light at 180°C.

**Patentansprüche**

**1.** Vorrichtung zur Überwachung der Qualität gereinigten Wassers, insbesondere für biologische Kläranlagen und dergleichen, umfassend

- eine Meßvorrichtung (2), bestehend aus einer Meßsonde mit einer Beleuchtungsvorrichtung (3) zur Beleuchtung des zu überwachenden Wassers, einer Reflexionsvorrichtung (15) zur Reflexion des von der Beleuchtungseinrichtung (3) ausgestrahlten Lichts und einer Empfangsvorrichtung (4) zum Empfang des von der Reflexionseinrichtung (15) reflektierten Lichts,
- einer Lesevorrichtung (5) zur Erzeugung eines Signals, welches proportional ist zu der Differenz zwischen einem Lichtsignal, welches von der Beleuchtungsvorrichtung emittiert wird und dem Lichtsignal, welches von der Empfangsvorrichtung nach Reflexion durch die Reflexionsvorrichtung empfangen wird, wobei die Lesevorrichtung (5) an die Beleuchtungsvorrichtung (3) und die Empfangsvorrichtung (4) angeschlossen ist, und
- eine Überwachungworrichtung (7), welche an die Lesevorrichtung angeschlossen ist und das von der Lesevorrichtung erzeugte Signal verarbeiten kann, um einen Schätzwert der Konzentration schädlicher Substanzen in dem zu überwachenden Wasser zu berechnen, um die Wahrscheinlichkeiten anzugeben, daß die entsprechenden tatsächlichen Konzentrationswerte für diese schädlichen Substanzen unterhalb eines vorbestimmten Grenzwertes liegen und anzuzeigen, falls diese Wahrscheinlichkeiten geringer oder höher sind als vorbestimmte Wahrscheinlichkeitsgrenzwerte.

**2.** Vorrichtung nach Anspruch 1, **dadurch gekennzeichnet,** daß die Überwachungsvorrichtung (7) einen Analog-Digital-Konverter (6) und eine Prozessoreinheit (8) aufweist, wobei der Konverter (6) das von der Lesevorrichtung (5) kommende Signal in ein Signal konvertiert, welches von der Prozessoreinheit (8) gelesen werden kann.

**3.** Vorrichtung nach Anspruch 2, **dadurch gekennzeichnet,** daß die Prozessoreinheit (8, 13) das von dem Konverter (6) erzeugte Signal verarbeiten kann unter Verwendung von Gleichungen, welche durch Korrelation von Werten dieses Signals erhalten werden, welche von der Lesevorrichtung (5) erzeugt werden mit Konzentrationsdaten, welche durch Laboranalysen erhalten werden.

4. Vorrichtung nach Anspruch 3, **dadurch gekennzeichnet,** daß die von der Prozessoreinheit (8, 13) benutzten Gleichungen kontinuierlich mit neuen, durch Laboranalysen erhaltenen Daten, aktualisiert werden.

5. Vorrichtung nach einem oder mehreren der voranstehenden Ansprüche, **dadurch gekennzeichnet,** daß sie Visualisierungsmittel (11) aufweist, welche der Überwachungsvorrichtung (7) zugeordnet sind.

6. Vorrichtung nach Anspruch 5, dadurch gekennzeichnet, daß die Visualisierungsmittel (11) ein Aufnahmegerät (21) mit einer Anzeigevorrichtung (22) umfassen.

7. Vorrichtung nach einem der Ansprüche 5 oder 6, **dadurch gekennzeichnet,** daß die Visualisierungsmittel (11) einen ersten Diodenbereich (20b) und einen zweiten Diodenbereich (20a) aufweisen, wobei jede Diode dieser Bereiche einer Substanz entspricht, die sich in dem zu überwachenden Wasser befindet, jede Diode des ersten Bereichs (20b) visualisieren kann, daß für diese Substanz die Wahrscheinlichkeit, daß die tatsächliche Konzentration innerhalb eines gegebenen Grenzwertes liegt eine vorbestimmte Wahrscheinlichkeitsgrenze überschreitet, und jede Diode des zweiten Bereichs (20a) visualisieren kann, daß für diese Substanz die Wahrscheinlichkeit, daß die tatsächliche Konzentration innerhalb eines gegebenen Grenzwertes liegt geringer ist, als ein vorbestimmter Wahrscheinlichkeitsgrenzwert.

8. Vorrichtung nach einem oder mehreren der voranstehenden Ansprüche, **dadurch gekennzeichnet,** daß die Meßvorrichtung (2) sich innerhalb einer Kammer (16) befindet, welche in das zu überwachende Wasser eingelassen werden kann, die Kammer mindestens eine Einlaßöffnung (18) und eine Auslaßöffnung (17) aufweist und diese Öffnungen den Kreislauf des zu überwachenden Wassers zwischen der Beleuchtungsvorrichtung (3), der Empfangworrichtung (4) und der Reflexionsvorrichtung (15) ermöglichen.

9. Vorrichtung nach einem oder mehreren der voranstehenden Ansprüche, **dadurch gekennzeichnet,** daß die Beleuchtungsvorrichtung (3) und die Empfangsvorrichtung (4) zum Empfang des reflektierten Lichts durch optische Fasern gebildet werden.

10. Vorrichtung nach einem oder mehreren der voranstehenden Ansprüche, **dadurch gekennzeichnet,** daß die Lesevorrichtung (5) eine Photozelle umfaßt.

11. Vorrichtung nach einem oder mehreren der voranstehenden Ansprüche, **dadurch gekennzeichnet,** daß die Reflexionsvorrichtung Licht bei 180°C reflektiert.

**Revendications**

1. Dispositif de contrôle de qualité de l'eau purifiée, en particulier pour installations de purification biologique et semblables, qui comprend :

   - des moyens de mesure (2) constitués d'une sonde de mesure comprenant un moyen (3) servant à éclairer ladite eau à contrôler, des moyens (15) servant à réfléchir la lumière émise par ledit moyen d'éclairage (3) et un moyen (4) destiné à recevoir la lumière réfléchie par lesdits moyens de réflexion,
   - un moyen de lecture (5) adapté pour générer un signal qui est proportionnel à la différence entre un signal lumineux émis par ledit moyen d'éclairage et le signal lumineux reçu par ledit moyen de réception après avoir été réfléchi par ledit moyen de réflexion, ledit moyen de lecture (5) étant relié audit moyen d'éclairage (3) et audit moyen de réception (4), et
   - des moyens de contrôle (7) connectés audit moyen de lecture et adaptés pour traiter le signal généré par ledit moyen de lecture pour calculer une estimation de la concentration de substances nocives dans l'eau à contrôler, pour indiquer les probabilités que les valeurs de concentration réelle correspondantes desdites substances nocives soient inférieures à un seuil prédéterminé et pour signaler si lesdites probabilités sont inférieures ou supérieures aux limites de probabilité sélectionnées.

2. Dispositif selon la revendication 1, caractérisé en ce que lesdits moyens de contrôle (7) comprennent un convertisseur analogique-numérique (6) et des moyens de traitement (8), ledit convertisseur (6) convertissant le signal provenant dudit moyen de lecture (5) en un signal qui peut être lu par lesdits moyens de traitement (8).

3. Dispositif selon la revendication 2, caractérisé en ce que lesdits moyens de traitement (8, 13) sont adaptés pour traiter ledit signal généré par le convertisseur (6) en utilisant des équations obtenues par la corrélation de valeurs dudit signal généré par ledit moyen de lecture (5) avec des données de concentration obtenues par des analyses de laboratoire.

4. Dispositif selon la revendication 3, caractérisé en ce que lesdites équations utilisées par lesdits moyens de traitement (8, 13) sont remises à jour de manière continue avec de nouvelles données obtenues par des analyses de laboratoire.

5. Dispositif selon l'une quelconque des revendications précédentes, caractérisé en ce qu'il comprend des moyens de visualisation (11) qui sont associés auxdits moyens de contrôle (7).

6. Dispositif selon la revendication 5, caractérisé en ce que lesdits moyens de visualisation (11) comprennent un enregistreur (21) avec un dispositif d'affichage (22).

7. Dispositif selon la revendication 5 ou 6, caractérisé en ce que lesdits moyens de visualisation (11) comprennent un premier ensemble de diodes (20b) et un second ensemble (20a) de diodes, chaque diode desdits ensembles correspondant à une substance qui est présente dans l'eau à contrôler, chacune desdites diodes dudit premier ensemble (20b) étant adaptée pour visualiser le fait que pour ladite substance ladite probabilité pour que la concentration réelle soit en deçà d'un seuil donné dépasse une limite de probabilité présélectionnée, et chacune desdites diodes dudit second ensemble (20a) étant adaptée pour visualiser le fait que pour ladite substance ladite probabilité pour que la concentration réelle soit en deçà d'un seuil donné est inférieure à une limite de probabilité présélectionnée.

8. Dispositif selon l'une quelconque des revendications précédentes, caractérisé en ce que lesdits moyens de mesure (2) sont insérés dans une chambre (16) adaptée pour être immergée dans ladite eau à contrôler, ladite chambre comprenant au moins une ouverture d'entrée (18) et une ouverture de sortie (17), lesdites ouvertures permettant la circulation de ladite eau à contrôler entre lesdits moyens d'éclairage (3) et de réception (4) et lesdits moyens de réflexion (15).

9. Dispositif selon l'une quelconque des revendications précédentes, caractérisé en ce que lesdits moyens d'éclairage (3) et ledit moyen (4) destiné à recevoir la lumière réfléchie sont constitués de fibres optiques.

10. Dispositif selon l'une quelconque des revendications précédentes, caractérisé en ce que ledit moyen de lecture (5) comprend une cellule photoélectrique.

11. Dispositif selon l'une quelconque des revendications précédentes, caractérisé en ce que lesdits moyens de réflexion réfléchissent la lumière à 180°.

Fig.1

Fig.3

Fig.2